# EUROPÄISCHE PATENTSCHRIFT

(11) **EP 1 383 523 B1**
(45) Veröffentlichungstag und Bekanntmachung des Hinweises auf die Patenterteilung: **07.06.2006**
(21) Anmeldenummer: 02727342.4
(22) Anmeldetag: 01.03.2002
(51) Int. Cl.: A61K 36/00, A61P 3/10

(54) **KOMBINATIONSPRÄPARAT ZUR BEHANDLUNG DES DIABETES MELLITUS**
AGENT FOR TREATING DIABETES MELLITUS
AGENT POUR TRAITER LE DIABETE SUCRE

(30) Priorität: 02.03.2001 DE 10110124; 05.04.2001 DE 10117185; 02.08.2001 DE 10137459
(43) Veröffentlichungstag der Anmeldung: 28.01.2004
(73) Patentinhaber: Lichtwer Pharma AG, 13435 Berlin (DE)
(72) Erfinder: TREPEL, Friedrich, 76135 Karlsruhe (DE); SCHNEIDER, Walter, 59823 Arnsberg (DE)
(74) Vertreter: Basfeld, Rainer
(86) Internationale Anmeldenummer: PCT/EP2002/002232
(87) Internationale Veröffentlichungsnummer: WO 2002/069987

(56) Entgegenhaltungen:
- DAVID J. A. JENKINS ET AL: "Manipulation of Gut Hormone Response to Food by Soluble Fiber and alpha-Glucosidase Inhibition" THE AMERICAN JOURNAL OF GASTROENTEROLOGY, Bd. 83, Nr. 4, 1988, Seiten 393-397, XP001113102
- F. REQUEJO ET AL: "Effects of alpha-Glucosidase Inhibition and Viscous Fibre on Diabetic Control and Postprandial Gut Hormone Responses" DIABETIC MEDICINE, Bd. 7, 1990, Seiten 515-520, XP001113101
- NELSON R W ET AL: "EFFECTS OF DIETARY FIBER SUPPLEMENTATION ON GLYCEMIC CONTROL IN DOGS WITH ALLOXAN-INDUCED DIABETES MELLITUS" AMERICAN JOURNAL OF VETERINARY RESEARCH, XX, XX, Bd. 52, Nr. 12, 1. Dezember 1991 (1991-12-01), Seiten 2060-2066, XP002072419 ISSN: 0002-9645
- W. CASPARY ET AL: "Effects of combined use of dietary fibre and acarbose on postcrandial blood glucose, IRI, GIP and intestinal H2-production" HEPATO-GASTROENTEROLOGY, Bd. 27/Suppl., 1980, Seite 364 (E44.7) XP002214098
- J. G¹RARD ET AL: "Fibres diététiques et acarbose dans le traitemant du diabète sucré" M¹DECINE ET HYGIÈNE, Bd. 39, Nr. 1434, 31. August 1981 (1981-08-31), Seiten 2674, 2677-2678, 2680, XP002214099
- VANNASAENG S ET AL: "EFFECTS OF ALPHA-GLUCOSIDASE INHIBITOR (ACARBOSE) COMBINED WITH SULFONYLUREA OR SULFONYLUREA AND METFORMIN IN TREATMENT OF NON-INSULIN-DEPENDENT DIABETES MELLITUS" CHOT MAI HET THANG PHAET - JOURNAL OF THE MEDICAL ASSOCIATION OF THAILAND, MEDICAL ASSOCIATION OF THAILAND, TH, Bd. 78, 1995, Seiten 578-585, XP000881905 ISSN: 0125-2208

## Beschreibung

Kohlenhydrate sind ein lebenswichtiger pflanzlicher Nahrungsbestandteil. Hauptnahrungsmittel ist die Stärke (Amylose und Amylopectin), ein aus zahlreichen Traubenzuckermolekülen aufgebautes Polysaccharid. Dazu kommen die Zucker in Form von Disacchariden wie der Haushaltszucker Saccharose (Sucrose), bestehend aus einem Molekül Traubenzucker und einem Molekül Fruchtzucker und schließlich Monosaccharide wie die Glucose, die nur aus einem Molekül Traubenzucker besteht.
Glucose wird nach der Magenpassage im Dünndarm innerhalb weniger Minuten vollständig aufgenommen (resorbiert). Saccharose muss im Dünndarm einmal gespalten werden (zu Glucose und Fruktose) und wird daher etwa 10 Minuten später zur Resorption verfügbar. Stärke wird vielmals gespalten, wobei die frei werdende Glucose erst nach 20-60 Minuten resorbiert wird. Die Spaltung der Kohlenhydrate erfolgt durch Enzyme (Glucosidasen und Amylasen).
Die im Rahmen der Verdauungsvorgänge resorbierten Kohlenhydrate werden durch den Blutkreislauf in Form von Traubenzucker als "Blutzucker" (Blutglucose) zu den Körperzellen transportiert. Nach Aufnahme von Kohlenhydraten mit der Nahrung steigt beim Gesunden der Blutzucker nach ½ - 1 Stunde leicht, beim Zuckerkranken dagegen viele Stunden stark an. Der Nüchternblutzucker beträgt beim Gesunden durchschnittlich 80-110 mg/dl und steigt nach einer kohlenhydrathaltigen Mahlzeit maximal bis 160 mg/dl. Beim Zuckerkranken variieren die Nüchternwerte zwischen 100 und 300 mg/dl. Nach einer Mahlzeit steigt der Blutzucker um weitere 80-200 mg/dl. Eine Blutzuckerkonzentration von über 160 mg/dl ist schädlich. Deshalb ist es Ziel jeder Diabetestherapie, den Blutzucker dauerhaft unter diesen Wert zu senken.

Bei der Zuckerkrankheit (Diabetes mellitus) ist die Funktion des in der Bauchspeicheldrüse gebildeten blutzuckerregulierenden Hormons Insulin gestört. Während der Gesunde den Blutzucker nach jeder Kohlenhydratmahlzeit mit Hilfe seines Insulins rasch wieder auf das normale Niveau um 100 mg/dl einstellt, gelingt das dem Diabetiker nicht. Man unterscheidet zwei Diabetesarten. Beim in der Jugend beginnenden Typ I-Diabetes fehlt das Insulin vollständig. Dagegen wird beim in der zweiten Lebenshälfte entstehenden Typ II-Diabetes noch Insulin gebildet. Es wirkt jedoch abgeschwächt und verzögert. Beim Typ II-Diabetes, der über 90% aller Zuckerkranken ausmacht, entwickelt sich frühzeitig eine allmählich abnehmende Reaktion der Körperzellen auf Insulin. Diese Insulinresistenz führt zu einer zunehmenden Verschlechterung des Krankheitsverlaufs und sollte durch geeignete Behandlungsmaßnahmen unbedingt verhindert werden. Der Diabetes mellitus ist in den Wohlstandsländern neben den Herz-Kreislauferkrankungen die bedeutendste Volkskrankheit. In Deutschland sind etwa 6 Millionen Menschen betroffen (4 Millionen im fortgeschrittenen, 2 Millionen im Frühstadium). Weltweit dürfte die Zahl der Zuckerkranken bei 200 Millionen liegen. Die Krankheitsdauer des Typ II-Diabetes beträgt durchschnittlich 20 Jahre, die krankheitsbedingte Lebensverkürzung etwa 6 Jahre.

Abgesehen von einer zuckerfreien und kohlehhydratkontrollierten Diät existieren fünf medikamentöse Behandlungsverfahren:
1. Insulininjektion (bei Typ I obligat, bei Typ II in den späteren Stadien)
2. Antidiabetische Tabletten vom Sulfonylharnstofftyp (z.B. Glibenclamid) und vom Glinid-Typ, die beim Typ II-Diabetes die verbliebenen Insulinreserven mobilisieren.
3. Antidiabetische Tabletten vom Biguanidtyp (z.B. Metformin), die beim Typ II-Diabetiker eine unphysiologische Glucoseverwertung bewirken und dadurch den Blutzucker senken.
4. Antidiabetische Tabletten vom Glitazontyp, die die Körperzellen wieder empfindlicher gegen Insulin machen und daher beim Typ II-Diabetes blutzuckersenkend wirken können.
5. Antidiabetische Tabletten, die durch Hemmung der kohlenhydratspaltenden Glucosidasen die Glucoseaufnahme im Dünndarm herabsetzen und damit den Blutzuckeranstieg nach dem Essen vermindern. Diese Alpha-Glucosidasehemmer können bei beiden Diabetestypen angewandt werden.

### Stellenwert der Therapieoptionen

### Zu 1:

Insulin ist das am stärksten blutzuckersenkende Medikament und stellt die überlebensnotwendige Grundtherapie des Typ I-Diabetes dar. Beim Typ II ist seine Anwendung auf die späteren Stadien beschränkt, in denen die bisher zur Verfügung stehenden antidiabetisclien Tabletten nicht mehr ausreichend wirken. Neben seinen unverzichtbaren positiven Wirkungen hat Insulin Nebenwirkungen (lebensgefährliche Unterzuckerungen, Förderung der Arteriosklerose, Förderung der Gewichtszunahme).

### Zu 2:

Sulfonylharnstoffe und Glinide wirken anfangs sehr gut blutzuckersenkend, verlieren aber nach wenigen Jahren wegen der Erschöpfung der Insulinvorräte ihren Effekt, fördern die Gewichtszunahme und haben wie Insulin nicht selten schwere Nebenwirkungen (z.B. Unterzuckerungen).

### Zu 3:

Biguanide wirken mäßig stark blutzuckersenkend, dürfen aber bei den vielen Patienten mit diabetischen Spätschäden oder einem Alter über 70 Jahren nicht angewendet werden und haben häufig Nebenwirkungen, darunter schwerwiegende.

### Zu 4:

Glitazone sind nur in Kombination mit Sulfonylharnstoff- oder Biguanidpräparaten zur Therapie zugelassen, weil ihre Wirkung erst nach 8-12wöchiger Behandlung einsetzt und relativ schwach ist. Als gerade beim Typ II-Diabetiker unerwünschte Nebenwirkung führen Glitazone zur Gewichtszunahme.

### Zu 5:

Die Alpha-Glucosidasehemmer mindern zwar zuverlässig den Blutzuckeranstieg nach dem Essen, haben aber bei etwa der Hälfte der behandelten Patienten lästige Nebenwirkungen von Seiten des Magen-Darmtrakts, sodass hierdurch die Behandlung in ausreichender Dosis eingeschränkt ist.

Als Schlussfolgerung ist festzuhalten: Alle fünf Therapieprinzipien beim Typ II-Diabetes sind wirksam, aber substanzspezifisch (Sulfonylharnstoffe, Biguanide) oder in therapeutisch optimaler Dosis (Insulin, Sulfonylharnstoffe, Biguanide, Alpha-Glucosidasehemmer) mit erheblichen Nebenwirkungen belastet. Die als langfristiges Therapieziel notwendige Schonung der körpereignen Insulinreserven wird nur durch Insulin, Biguanide und Alpha-Glucosidasehemmer erreicht. Die unerwünschte Hyperinsulinämie wird nur durch Glitazone, Biguanide und Alpha-Glucosidasehemmer vermindert. Die wünschenswerteste Behandlung in den frühen und mittleren Stadien des Typ 11-Diabetes wäre zwar die Therapie mit Alpha-Glucosidasehemmern, weil sie bei mittelgradiger Wirksamkeit keine gefährlichen Nebenwirkungen aufweist, aber die meisten Patienten tolerieren die lästigen Begleiterscheinungen (Blähsucht, Durchfallneigung) nicht.

Die WO 93/16 605 beschreibt die therapeutische Verwendung von unverdaulichen Faserstoffpräparaten mit einem Anteil von 25-60 % Rübenballaststoff (entsprechend 1,25-12 g/Tag) zur Senkung des erhöhten Blutdrucks beim Menschen. Die zugrundeliegenden Daten wurden an Blutdruckpatienten erarbeitet, die keinen Diabetes hatten. Im Zuge umfassender Datenerhebung über die Nieren- und Schilddrüsenfunktion sowie den Fettstoffwechsel dieser Studienteilnehmer wurde auch der Nüchternblutzucker und die Nüchternkonzentration des Insulins im Blutserum sowie der indirekte integrale Blutzuckermesswert HbA1C gemessen. Der Nüchternblutzucker blieb nach mehrmonatiger Ballastbehandlung unverändert, dagegen sank der Insulinspiegel. Der HbA1C-Wert nahm in der Ballastgruppe ebenso ab wie in der Kontrollgruppe. Aus diesen Ergebnissen haben die Autoren keinen eindeutigen Effekt von (Rüben)ballaststoff auf den Kohlehydratstoffwechsel von Nicht-Diabetikern abgeleitet.

Ballaststoffe wurden vor 20 Jahren in die Diabetestherapie eingeführt. Ballaststoffe sind nicht-verdauliche pflanzliche Substanzen, die die Nahrung begleiten. Als wirksam erwiesen sich bislang vor allem wasserlösliche Ballaststoffe wie Guar, Pektin, Psyllium und Betaglucane. Diese Stoffe haben jedoch alle in ausreichender Dosis therapieeinschränkende Eigenschaften wie Störungen der Darmtätigkeit oder Geschmacks- und Schluckbeeinträchtigungen. Im Gegenatz zu den löslichen wurden die wasserunlöslichen Ballaststoffe bei der Therapie des menschlichen Diabetes bislang nicht systematisch untersucht. Wasserunlösliche Ballaststoffe sind die Polysaccharide Cellulose, Hemicellulose (Pentosane) und das Phenylpropan-Polymer Lignin.

D. J. A. Jenkins at al. beschreiben in The American Journal of Gastroenterology, Band 83, 4, 1988, Seiten 393 bis 397 den Einfluss einer Kombination aus Guar und dem α-Glucosidasehemmer Acarbose bei einer Reihe von untersuchten Testpersonen. Guar ist ein weit überwiegend (85 %) wasserlöslicher Ballaststoff und besteht zu 65 % aus Mannose und zu 35 % aus Galaktose. Es wurden nur Kurzzeitversuche durchgeführt und bei den untersuchten Personen handelt es sich nicht um Diabetiker. Es wurde festgestellt, dass bei den meisten Versuchspersonen innerhalb der folgenden 22 Stunden die typischen lästigen Nebenwirkungen der Acarbose auftraten. Bei einer Langzeitanwendung, wie sie in der Diabetestherapie notwendig ist, sind die gesteigerten kombinierten Nebenwirkungen der beiden Substanzen (Guar und Acarbose) nicht akzeptabel.

F. Requejo et al beschreiben in Diabetic Medicino, Vol. 7, 1998, Seiten 515 bis 520 die Wirkung einer Kombination von Alpha-Glucosidasehemmern mit Guar, wobei ein Dutzend Diabetes Typ II-Patienten untersucht wurden. Bei diesen Versuchen handelt es sich um Beobachtungen über einen längeren Zeitraum und es wurde festgestellt, dass sich die Nebenwirkungen der einzelnen Substanzen verstärken. Die gastrointestinalen Nebenwirkungen verdoppelten sich gegenüber der Einzeltherapie mit dem Glucosidasehemmer und verneunfachten sich gegenüber der Anwendung von Guar allein. Wasserlösliche Ballaststoffe wie Guar behindern durch ihre Viskosität die Glucoseresorption im Darm. Die nichtresorbierte Glucose wird durch Darmbakterien zersetzt, was die Nebenwirkungen auslöst.

R. W. Nelson et al beschreiben in American Journal of Veterinary Research, Band 52 (12), 1991, Seiten 2060 bis 2066 Effekte einer Diät mit hohem Anteil unlöslicher Fasern in einer Untersuchung an Hunden, die an dem insulinabhängigen Diabetes Typ 1 erkrankt waren. Es werden Diäten mit Kombinationen unlöslicher und löslicher Ballaststoffe untersucht. Die beiden verabreichten Diäten sind nur quantitativ und nicht qualitativ verschieden und daher nicht so aussagekräftig wie von den Autoren angenommen. Die IF-Diät (unlösliche Ballaststoffe) und die SF-Diät (lösliche Ballaststoffe) senkten verglichen mit der ballaststoffarmen Diät den Blutzucker in gleicher Stärke. Die Ergebnisse wurden an Hunden erarbeitet, die an künstlich erzeugtem Typ I-Diabetes litten. Die Ergebnisse solcher Untersuchungen sind auf den Menschen nicht ohne weiteres übertragbar.

Die vorliegende Erfindung beschreibt Wege, wie der Blutzuckeranstieg nach dem Essen zuverlässig, stark und ohne relevante Nebenwirkungen oder Begleiterscheinungen gesenkt wird und langfristige Besserungen des Krankheitsverlaufs erreicht werden.

Aufgabe der vorliegenden Erfindung ist es, ein Mittel zur Behandlung des Diabetes mellitus zur Verfügung zu stellen, welches eine gute Wirksamkeit hat, ohne dass die vorgenannten Nebenwirkungen auftreten.

Dieses Ziel wird durch ein Mittel zur Behandlung des Diabetes mellitus mit den Merkmalen eines der Patentansprüche 1, 5, 14 oder 24 erreicht.

Das erfindungsgemäße Mittel zur Behandlung des Diabetes mellitus zeichnet sich gemäß einer ersten Variante der Erfindung dadurch aus, dass es Rübenballaststoff als Wirkstoff enthält. Das erfindungsgemäße Mittel wird vorzugsweise zur Behandlung der Frühstadien des Diabetes mellitus Typ II eingesetzt.

Rübenballaststoff ist ein feinkörniger Schrot (Granulat) aus Zuckerrübenresten, dem sogenannten Zuckerrübenmark, nach vorheriger Gewinnung bzw. Abtrennung des Zuckers.

Seine Zusammensetzung ist z. B. Pektin 31 %, Pentosane 24 %, Cellulose 24 %, Lignin 4 %, Eiweiß 9 %, Asche (Mineralsalze) 4 %, Saccharose 4 % (Durchschnittswerte). Zuckerrübenreste dieser Art dienen vor allem als Viehfutterzusatz. In Form des beschriebenen Granulats wird Rübenballast auch als Diätzusatz für die menschliche Nahrung "zur Darmregulierung und cholesterinbewussten Ernährung" empfohlen. Rübenballaststoff wird von Zuckerfabriken hergestellt. Der Stoff ist direkt bei der Mühle oder in Reformhäusern zu beziehen. Er ist nicht apotheken- oder rezeptpflichtig, also bisher nicht als Heilmittel oder Medikament einzustufen.

Die Darreichung des Rübenballaststoffs erfolgt vorzugsweise in Form von Granulat, Pulver oder Keksen. Bevorzugt wird die Darreichungsform als Granulat, insbesondere als dragiertes Granulat, um das Schlucken des im Pulverzustand relativ trockenen Rübenballaststoffs dem Patienten zu erleichtern.

Im Rahmen der vorliegenden Erfindung haben sich Dosierungen bei Gabe des Rübenballaststoffs insbesondere als dragiertes Granulat in Einzeldosen von zwischen etwa 5 g bis etwa 20 g als vorteilhaft erwiesen. Bevorzugt sind Einzeldosen von zwischen etwa 10 g und etwa 20 g. Vorzugsweise erfolgt die Einnahme des Rübenballaststoffs vor oder zu Beginn einer Mahlzeit.

Durch die Untersuchungen im Rahmen der vorliegenden Erfindung konnte gezeigt werden, dass zur Therapie insbesondere der Frühstadien des Diabetes mellitus Typ II eine Kombination des Wirkstoffs Rübenballaststoff mit einem weiteren Wirkstoff ausgewählt aus Acarbose, Miglitol oder einem anderen Alphaglucosidasehemmer zu besonders guten Ergebnissen führt sowohl hinsichtlich der verbesserten Therapiewirkung durch die Wirkstoffkombination als auch im Hinblick auf wesentlich herabgesetzte Nebenwirkungen im Vergleich zu einer Gabe der Einzelwirkstoffe.

Nachstehend ist die Formel für Acarbose wiedergegeben:

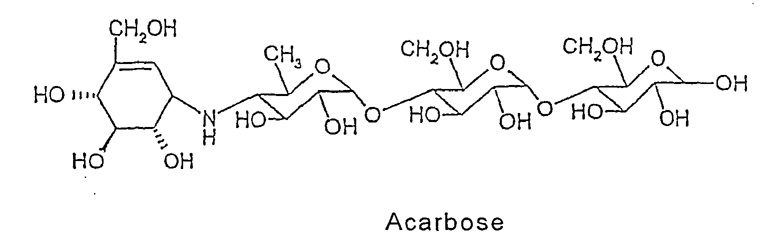

Nachstehend ist die Formel für Miglitol wiedergegeben:

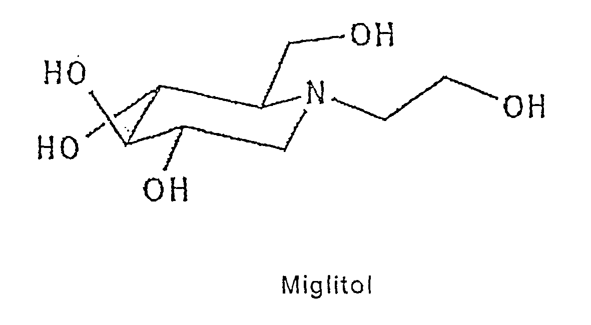

Neben Acarbose und Miglitol kommen z. B. Voglibose oder Emiglitat als Alphaglucosidasehemmer in Betracht.

Die Darreichung einer solchen Kombination gemäß der Erfindung kann als Mischpräparat erfolgen, welches gleichzeitig Rübenballaststoff und/oder Acarbose, Miglitol bzw. andere Alphaglucosidasehemmer enthält. Ebensogut ist die getrennte Einnahme von Rübenballaststoff und Acarbose oder Miglitol möglich. Die Einnahme beider Medikamente sollte möglichst in unmittelbarem zeitlichen Zusammenhang erfolgen. Auch in diesen Fällen kann der Rübenballaststoff in unterschiedlicher Darreichungsform appliziert werden, wobei die Einnahme als Granulat, insbesondere als dragiertes Granulat bevorzugt ist. Die Einnahme des zweiten Wirkstoffs Acarbose und/oder Miglitol bzw. eines anderen Alphaglucosidasehemmers kann beispielsweise in Tablettenform erfolgen.

In der Regel ist die Menge des Wirkstoffs Acarbose/Miglitol in dem erfindungsgemäßen Medikament bzw. in der Medikamentenkombination wesentlich geringer als die eingenommene Menge des Rübenballaststoffs. Da beispielsweise Mengen von 5 bis 30 g, vorzugsweise etwa 10 bis etwa 20 g Rübenballaststoff als besonders geeignet angesehen werden und Mengen von etwa 20 bis etwa 200 mg Acarbose und/oder Miglitol, vorzugsweise Mengen von etwa 50 mg bis etwa 100 mg Acarbose/Miglitol pro Dosis liegt die Menge an Rübenballaststoff bei in der Regel etwa dem 25-fachen bis 1.000-fachen der Menge an Acarbose bzw. Miglitol, wobei Mengenverhältnisse von etwa dem 400-fachen bis 100-fachen an Rübenballaststoff bezogen auf den oder die anderen Wirkstoffe in den bevorzugten Bereichen gegeben sind.

Es konnten weitere Ballaststoffe und Alpha-Glucosidasehemmer untersucht werden, wobei wesentliche Erkenntnisse über die wirksamen Bestandteile der Ballaststoffe im Hinblick auf eine Verwendung zur Behandlung des Diabetes mellitus gewonnen wurden.

Im Rahmen einer Variante der Erfindung hat sich insbesondere die Kombination pflanzlicher Ballaststoffe mit Alpha-Glucosidasehemmern als vorteilhaft erwiesen.

Im Rahmen einer Weiterbildung der Erfindung ist die Verwendung wenigstens eines Ballaststoffs mit einem hohen Anteil unlöslicher Faserstoffe besonders bevorzugt. Der Anteil an unlöslichen Faserstoffen, der in einem solchen Ballaststoff enthalten ist, kann beispielsweise mehr als 20 g pro 100 g Ballaststoff betragen.

Die zu verabreichende Einzeldosis enthält im Rahmen einer bevorzugten Weiterbildung der Erfindung vorzugsweise etwa 2,5 bis 10 g unlösliche Fasern. Besonders bevorzugt ist die Einnahme des erfindungsgemäßen Mittels mit den Hauptmahlzeiten, beispielsweise 1 bis 3 mal täglich.

Vorzugsweise erfolgt die Einnahme eines erfindungsgemäßen Mittels in Kombination mit einem der Alpha-Glucosidasehemmer Acarbose, Miglitol, Voglibose oder Emiglitat oder ähnlichen Stoffen mit vergleichbaren Eigenschaften und ähnlicher chemischer Konstitution. Besonders bevorzugt ist die Einnahme des Ballaststoffs in Kombination mit wenigstens einem der genannten Alpha-Glucosidasehemmer oder unmittelbar vor oder nach dessen Einnahme.

Die im Rahmen der vorliegenden Erfindung durchgeführten Untersuchungen führten unter anderem zu der Erkenntnis, dass andere pflanzliche Ballaststoffe eine ähnliche blutzuckersenkende Wirkung zeigen wie Rübenballaststoff, und dass einige Ballaststoffe ähnlich wie Rübenballaststoff die Wirkung von Alpha-Glucosidasehemmern verstärken. Eine gute Wirkung zeigte dabei beispielsweise Leinsamen, wobei jedoch festgestellt wurde, dass sich ein regelmäßiger Effekt bei Leinsamen allein erst nach der Entfettung nachweisen lässt. Es wurde weiterhin festgestellt, dass manche Ballaststoffe im Zusammenhang mit bestimmten Alpha-Glucosidasehemmern die genannte blutzuckersenkende Wirkung nicht zeigen.

Umfangreiche weitere Versuche der Anmelder haben zu der überraschenden Erkenntnis geführt, dass die Einnahme eines Cellulose enthaltenden Präparats, welches vorzugsweise die Cellulose in reiner Form ohne die sonstigen im Rübenballaststoff oder anderen pflanzlichen Ballaststoffen enthaltenen Substanzen enthält, bei der Behandlung des Diabetes zu besonders guten Ergebnissen führt. Als Ergebnis dieser Versuche kann davon ausgegangen werden, dass der antidiabetische Effekt auf die Cellulose zurückzuführen ist, die auch in unlöslichen Ballaststofffasergemischen der zuvor genannten Art enthalten ist.
Im Gegensatz zu dem zuvor genannten Rübenballaststoff handelt es sich bei Cellulose um ein fast geschmacksneutrales Mittel, welches z. B. in Pulverform in Wasser, Joghurt oder ähnlichen Lebensmitteln verabreicht leicht geschluckt werden kann und wie die Versuche der Anmelder gezeigt haben keine Nebenwirkungen oder unerwünschte Begleitwirkungen aufweist. Die Einnahme ist daher für den Patienten noch einfacher als bei Rübenballaststoffpräparaten.
Das für das erfindungsgemäße Mittel verwendete Präparat kann mehr oder weniger reine Cellulose enthalten, die kostengünstig herstellbar und gut verfügbar ist. Da die blutzuckersenkende Wirkung auch in den eingangs genannten Ballaststoffpräparaten offenbar auf die Cellulose zurückzuführen ist, ergibt sich der weitere Vorteil, dass bei Einnahme reiner Cellulose die Einnahme einer geringeren Menge des Mittels genügt. Die antidiabetische Wirkung der Cellulose ist ähnlich wie diejenige des zuvor untersuchten Rübenballasts bzw. des entfetteten Leinsamens. Ein erheblicher Vorteil der Cellulose liegt jedoch darin, dass sie in chemisch reiner Form verabreicht werden kann und damit ihre leichtere Standardisierbarkeit als Medikament gegeben ist und außerdem eine geringere Substanzmenge als therapeutische Dosis genügt. Die wirksame Einzeldosis der Cellulose kann im Vergleich zu den genannten komplexeren aus Pflanzen gewonnenen Ballaststoffen auf beispielsweise etwa die Hälfte bis etwa ein Zehntel der Substanzmenge reduziert werden. Dies erleichtert den Einsatz in der Diabetestherapie. Cellulose verstärkt den antidiabetischen Effekt der Alpha-Glucosidasehemmer.
Ein weiterer Vorteil der Verwendung von Cellulose in dem erfindungsgemäßen Mittel liegt darin, dass es sich um eine Substanz handelt, die in vielen Nahrungsmitteln vorliegt und daher für den menschlichen Körper völlig unbedenklich ist. Die Cellulose kann beispielsweise als Pulver oder auch als Kautablette in Verbindung mit einem Bindemittel konfektioniert werden. Da sich Cellulosepulver in nahrungsmittelüblichen Flüssigkeiten sehr gut und rasch suspendieren lässt, ist die Einnahme von Cellulosepulver für den Patienten bequem und die Handhabung ist sehr einfach. Die genannten bevorzugten Einzeldosen entsprechen etwa der Menge eines Esslöffels. Diese Menge kann in ein Getränk gegeben und eingerührt und so für den Patienten bequem verabreicht werden. Das erfindungsgemäße Mittel kann z. B. so konfektioniert werden, dass die jeweils für eine Einzeldosis notwendige Menge an Cellulosepulver z. B. in einem separaten Beutel verpackt einerseits und der Alpha-Glucosidasehemmer in Tablettenform andererseits in einer gemeinsamen größeren Verpackungseinheit in den Handel gelangt.
Untersuchungen im Rahmen der vorliegenden Erfindung haben ergeben, dass grundsätzlich anstelle der Verwendung von Cellulose auch die Einnahme eines Cellulosederivats in Betracht kommt. Solche Cellulosederivate können beispielsweise substituierte Cellulosen sein, die gegebenenfalls nicht pflanzliche. Herkunft sind, sondern synthetisch hergestellt werden. Beispielhaft sei hier die Methylcellulose genannt, die im Rahmen der vorliegenden Erfindung als Ballaststoff anstelle von Cellulose in Betracht kommt. Auch ein solches Cellulosederivat wird vorzugsweise mit einem Alpha-Glucosidasehemmer kombiniert, wobei beide Stoffe in Kombination in einem Präparat oder jeweils in getrennten Präparaten vorliegen können.

Im Rahmen der vorliegenden Erfindung hat sich entfetteter Leinsamen ebenfalls zur Therapie von Diabetes mellitus als geeignet erwiesen.

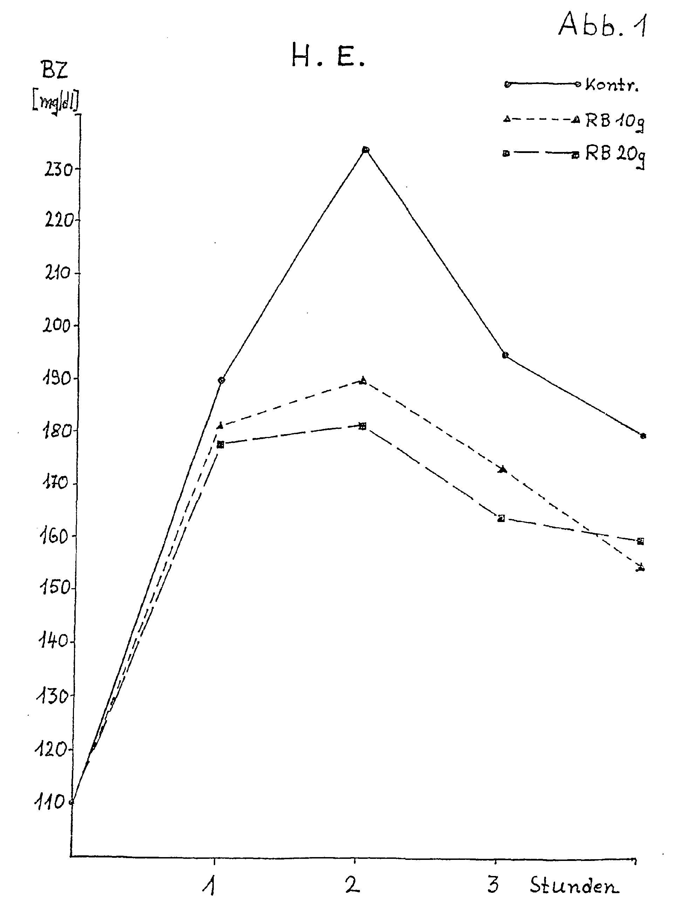

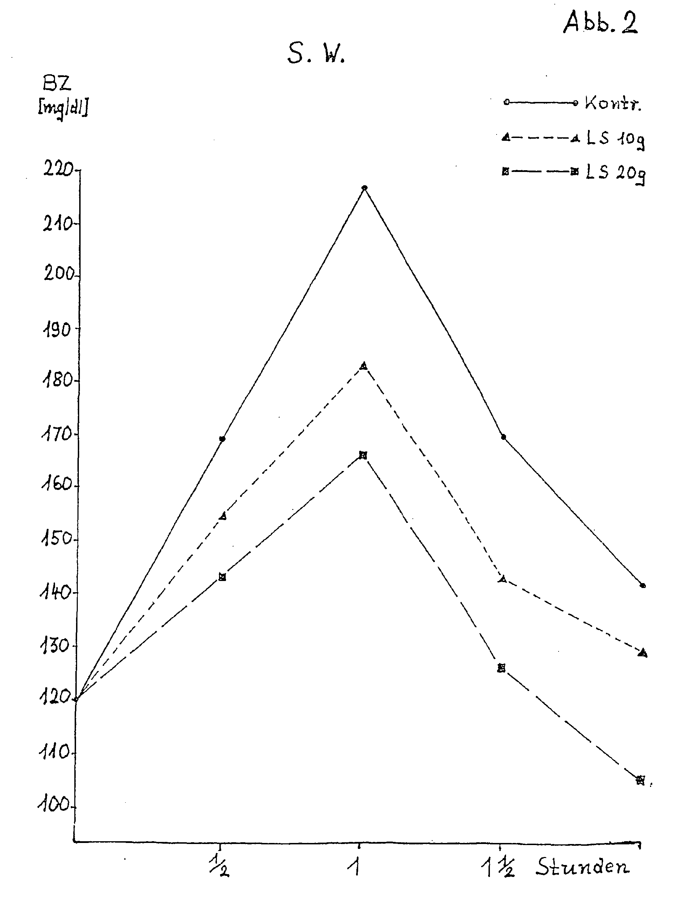

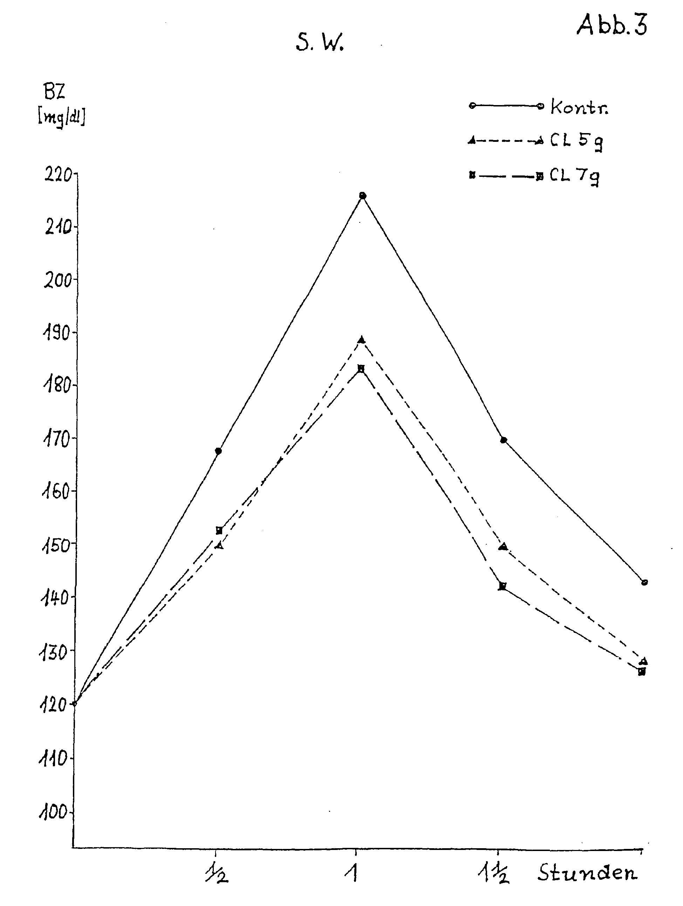

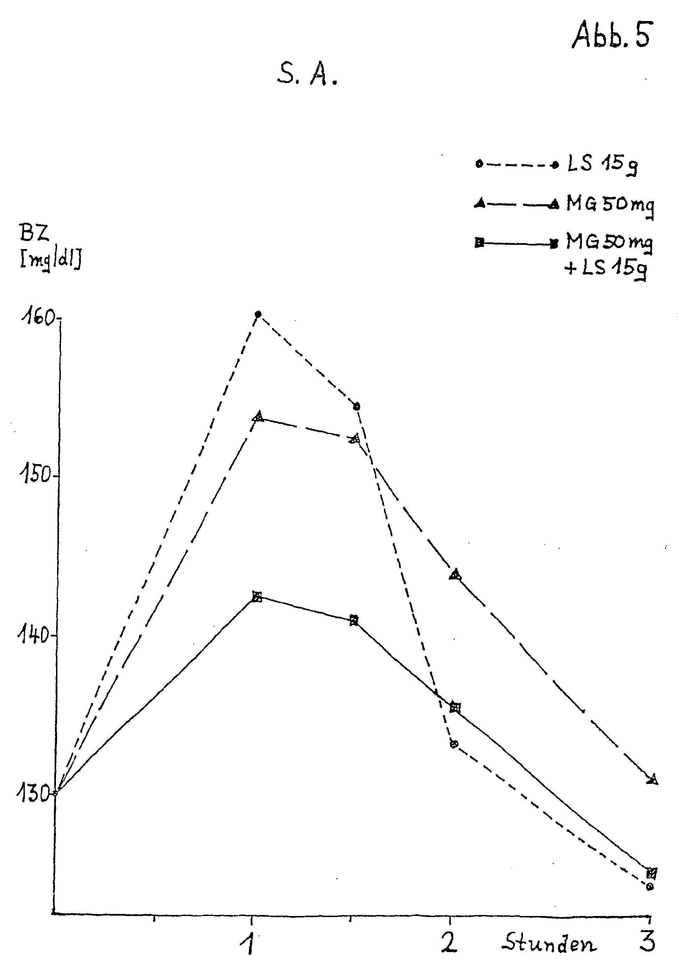

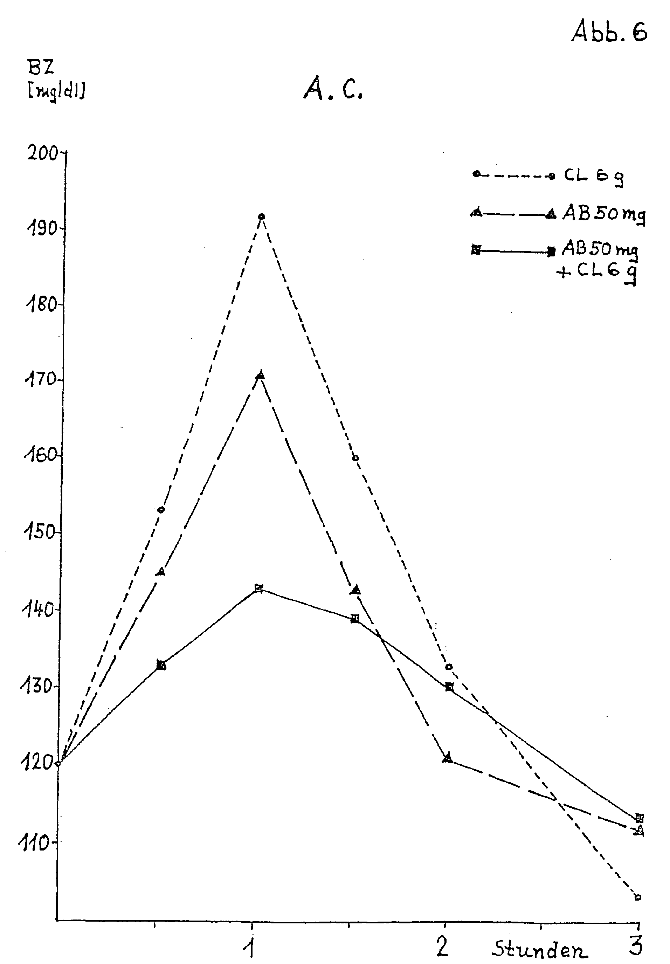

Nachfolgend wird die vorliegende Erfindung anhand von Ausführungsbeispielen näher beschrieben. Dabei wird in dem nachfolgenden Text auf die im Anhang beigefügten Tabellen 1 bis 7 Bezug genommen sowie auf die ebenfalls beiliegenden Abbildungen 1 bis 8. Eine Legende mit Erläuterungen zu den Abbildungen 1 bis 8 ist dem Anmeldetext ebenfalls als Anlage beigefügt.

Wir prüften Zuckerrübenballaststoff mit einem hohen Gehalt an Cellulose und Hemicellulose und relativ wenig Lignin (Verhältnis C : H : L = 6 : 6 : 1), Leinsamen mit hohem Gehalt an Hemicellulose (Verhältnis C: H: L = 2,5 : 5 : 1), Weizenkleie mit dem höchsten Ligningehalt (Verhältnis C : H : L = 2,6 : 2,7 : 1), reine Cellulose sowie als Vergleichssubstanz Methylcellulose, ein künstliches Cellulosederivat, das im Gegensatz zu Cellulose eine geringe Wasserlöslichkeit aufweist und in dieser Hinsicht zwischen den unlöslichen und löslichen Ballaststoffen steht. Die Zusammensetzung der Ballaststoffpräparate ist in Tabelle 1 zu entnehmen.

Rübenballaststoff (Hammermühle Diät GmbH, D-67489 Kirrweiler) ist ein Granulat (feinkörniger Schrot), das in Wasser suspendiert unangenehm schmeckt, aber in Joghurt gut zu schlucken ist. Durch Dragierung des Granulats wird eine noch bessere Einnahmequalität erzielt.
Leinsamen (käuflich im Reformhaus) wird als grobes Granulat ("geschroteter Leinsamen") verwendet und wird z. B. mit Aceton entfettet. Er kann mit Wasser oder angenehmer in Joghurt eingenommen werden und schmeckt nussartig.
Weizenkleie (käuflich im Reformhaus) ist ein flockiges Pulver, das wie Leinsamen eingenommen wird.
Cellulose (Synopharm GmbH, Postfach 1205, D-22882 Barsbüttel) ist im verwendeten Präparat ein feines mehlartiges Pulver, das auch mehlartig schmeckt und suspendiert in Wasser oder Joghurt leicht einzunehmen ist.

Methylcellulose (Synopharm GmbH, Postfach D-22882 Barsbüttel) ist ebenfalls ein mehlartiges Pulver, das in Wasser oder Joghurt zu klebrigen Klümpchen zusammenballt und sich weniger angenehm einnehmen lässt als Cellulose.

### Kurzzeitversuche mit Ballaststoffen

20 Typ II-Diabetiker erhielten nach morgendlicher Messung des Nüchternblutzuckers ein Testfrühstück (75 g Weißbrot, 20 g Butter, 40 g Marmelade oder als zuckerfreie Variante 75 g Weißbrot, 20 g Butter und 40 g Käse). An den Kontrolltagen wurden vor dem Testfrühstück 75ml Joghurt und an den Prüftagen die verschiedenen Ballaststoffe in 75ml Joghurt gegeben. Die Ballaststoffdosen betrugen 10-20 g (Rübenballaststoff, Leinsamen und Weizenkleie) oder 3-10 g (Cellulose und Methylcellulose). Nach dem Testfrühstück wurden die Blutzuckerwerte in ½- .bis 1-stündigem Abstand für 2-4 Stunden gemessen. Jeder Kurzzeitversuch wurde bei den einzelnen Versuchspersonen zwei- bis dreimal durchgeführt. Es werden die jeweiligen Durchschnittswerte angegeben. An den Versuchsvormittagen ließen die Patienten, sofern sie anderweitig antidiabetisch behandelt wurden, ihre entsprechenden Medikamente (Insulin, Sulfonylharnstoff- oder Biguanidpräparat) weg.

Grundlage für die Auswertung ist die grafische Darstellung der Blutzuckerwerte als Kurven. Die 1-2 Stunden dauernde Anstiegsphase des Blutzuckers wird planimetrisch bestimmmt und als Maß für die Untergrenze der Glucoseresorption betrachtet. In der ersten Stunde nach dem Testfrühstück dürfte unter körperlichen Quasi-Ruhebedingungen am Versuchsvormittag beim Diabetiker anders als beim Gesunden der Anstieg der Blutzuckerkurve die intestinale Glucoseaufnahme ins Blut fast unverfälscht widerspiegeln. Jenseits der ersten Stunde setzt auch bei den meisten Typ II-Diabetikern mehr oder weniger deutlich eine Insulinantwort ein, die den Blutzuckeranstieg abflacht oder umgekehrt, jedenfalls zu einer entsprechenden Unterschätzung der Glucoseresorption führt. In einzelnen Fällen erreicht die Kontrollkurve den Gipfelpunkt früher als die experimentellen Kurven. Dann wird die planimetrische Auswertung aller Kurven bis zum Erreichen des Gipfels der letzen Kurve ausgedehnt. Dies führt dazu, dass die ermittelten experimentellen Blutzuckersenkungen eher unter- als überschätzt werden. Aus praktischen Gründen vergleichen wir die Anstiegsphasen der Blutzuckerkurven unter den verschiedenen Versuchsbedingungen (Kontrolle, nach Ballaststoff, bei den späteren Versuchen auch nach Alpha-Glucosidasehemmern) miteinander und errechnen daraus die Grade der Blutzuckersenkung bezogen auf die Kontrollkurve. Die nach Behandlung mit Ballast- oder anderen Stoffen beobachtete Verringerung des Blutzuckeranstiegs entspricht also unter der Voraussetzung der zeitkonstanten Insulinantwort beim gleichen Patienten der erreichten Minderung der Glucoseresorption. Die statistische Auswertung, die aufgrund der Datenmengen nur bei den Kurzzeitversuchen möglich ist, erfolgt mit dem Student t-Test im paarweisen Vergleich.
Typische Beispiele werden in den Abbildungen 1-3 für Rübenballaststoff, Leinsamen und Cellulose gezeigt. Diese Ballaststoffe senkten den Blutzuckeranstieg nach Testmahlzeit mit einer gewissen Schwankungsbreite regelmäßig, im Durchschnitt um 30% (Tabelle 2). Die Senkung ist hochsignifikant (p<0.001). Ein Wirkungsunterschied zwischen Rübenballast, Leinsamen und Cellulose ist nicht zu erkennen. Dagegen ist Weizenkleie nicht eindeutig wirksam (Blutzuckersenkung um etwa 10%). In mehreren Fällen wurden verschiedene Dosen der Ballaststoffe getestet: Eine Halbierung der 20g-Dosis von Rübenballast oder Leinsamen brachte noch eine 80-90%ige Wirkung. Bei Cellulose wirkten 5, 6 und 7 g praktisch gleich. Drei und 10 g erzielten etwa 80% der maximalen Wirkung.

### Langzeitversuche mit Ballaststoffen

11 Typ II-Diabetiker erhielten vor den drei Hauptmahlzeiten jeweils 10 g Rübenballaststoff oder 5 g Cellulose. Der Blutzucker wurde 2 Stunden nach individuellem Frühstück täglich bestimmt, in den meisten Fällen auch der Nüchternblutzucker. Eine ballaststoff-freie Beobachtungsphase vor der Behandlungsperiode gilt als Kontrolle. Im Fall einer Vorbehandlung wurden die antidiabetischen Medikamente während der Zugabe von Ballaststoff unverändert beibehalten.

Tabelle 3 fasst die Ergebnisse zusammen: Nach alleiniger oder ergänzender Therapie mit Ballaststoffen wurden durchschnittlich der Nüchternblutzucker um 4 mg/dl und der Blutzucker nach dem Essen um 35 mg/dl gesenkt. Bei zwei Patienten, die genügend lange (4 Monate) beobachtet werden konnten, nahm der HbA1C-Wert um 0,6 bzw. 0,9% ab.

### Nebenwirkungen der Ballaststoffe

Unerwünschte Begleiteffekte traten bei den Kurzzeitversuchen nicht und während der Langzeitversuche bei 2 der 11 Patienten auf, jeweils mit Rübenballaststoff: Bei einer Patientin kam es jeden Morgen (nicht mittags und abends) nach Rübenballaststoffeinnahme zu einem 1-stündigen Sodbrennen, bei einem anderen Patienten verdoppelte sich die Stuhlfrequenz von 2- auf 4-mal täglich.

### Zusammensetzung und Bewertung der Ballaststoffversuche

Wasserunlösliche Ballaststoffe haben eine eindeutige und in jedem Einzelfall reproduzierbare antidiabetische Wirkung. Die wirksame Ballastfaser ist die Cellulose. Offenbar spielen die begleitenden Ballastfasern Hemicellulose und Lignin eine untergeordnete Rolle. Warum Weizenkleie trotz eines hohen Gehalts an Cellulose nur sehr schwach wirkt, ist unklar. Wahrscheinlich heben Begleitsubstanzen, z.B. das reichlich enthaltene Protein (15%), die antidiabetische Wirkung der Cellulose auf. Auch Methylcellulose hat offenbar nur einen schwachen Effekt. Da unlösliche Ballaststoffe wie Cellulose nicht im Dünndarm gespalten und resorbiert werden und ihr blutzuckersenkender Effekt trotzdem innerhalb 1 Stunde eintritt, müssen sie die Glucoseresorption innerhalb des Darms behindern. Dies geschieht wahrscheinlich durch eine teilweise Hemmung der stärkespaltenden Amylasen, wofür die große chemische Ähnlichkeit der Cellulose (beta-1 ,4-glycosidisches Glucosepolymer) und der Amylose (alpha-1,4-glycosidisches Glucosepolymer) spricht. Weitere Befunde bei unseren Kurzzeitversuchen stützen diese Annahme: Cellulose wirkte nach Gabe von reiner Saccharose (die unabhängig von der Amylase gespalten und resorbiert wird) nicht blutzuckersenkend.

Der blutzuckersenkende Effekt der Cellulose und der anderen untersuchten Ballaststoffpräparate ist mit durchschnittlich 30% relativ gering. Daher kommen die Ballaststoffe als Monotherapie nur für die Behandlung der Frühstadien des Typ II-Diabetes infrage. In den übrigen Krankheitsstadien lassen sie sich mit anderen Antidiabetica kombinieren.

### Kombination mit Alpha-Glucosidasehemmern

Die bisher bekannten Alpha-Glucosidasehemmer sind zuckerähnliche Stoffe, die im Dünndarm kompetitiv die Alphaglucosidasen inhibieren und damit die Spaltung der in der Nahrung enthaltenen Disaccharide und der beim Stärkeabbau entstehenden Oligosaccharide verlangsamen. Wir verwenden die bisher zur Therapie zugelassenen Substanzen Acarbose (Glucobay ®) und Miglitol (Diastabol ®). Es sind noch weitere Alpha-Glucosidasehemmer entwickelt worden, z.B. Voglibose und Emiglitat, die alle prinzipiell gleich blutzuckersenkend wirken. Hier wird geprüft, ob unlösliche Ballaststoffe den blutzuckersenkenden Effekt von Acarbose und Miglitol modifizieren.

### Kurzzeitversuche mit Alpha-Glucosidasehemmern und Ballaststoffen

21 Patienten wurden untersucht: je 10 erhielten Acarbose und Miglitol, 1 Patient zuerst Acarbose und dann Miglitol. Die verwendete Einzeldosis betrug 25-100 mg. Als Ballaststoffe dienten Rübenballast (10-20 g), entfetteter Leinsamen (8-15 g), Cellulose (3-10 g) und Methylcellulose (5-6 g).
Unmittelbar vor den oben beschriebenen Testmahlzeiten erhielten die Versuchspersonen entweder a) 75 ml Joghurt (Kontrollversuch) oder b) Ballaststoff in Joghurt (Ballastkurve) oder c) eine Tablette Acarbose bzw. Miglitol und danach 75 ml Joghurt (Alpha-GH-Kurve) oder d) dieses Medikament 5 Minuten vor einer Ballaststoffeinnahme in Joghurt (Ballast- plus Alpha-GH-Kurve). Wie beschrieben, wurden vor und während 3 Stunden nach der Testmahlzeit die Blutzuckerwerte bestimmt.

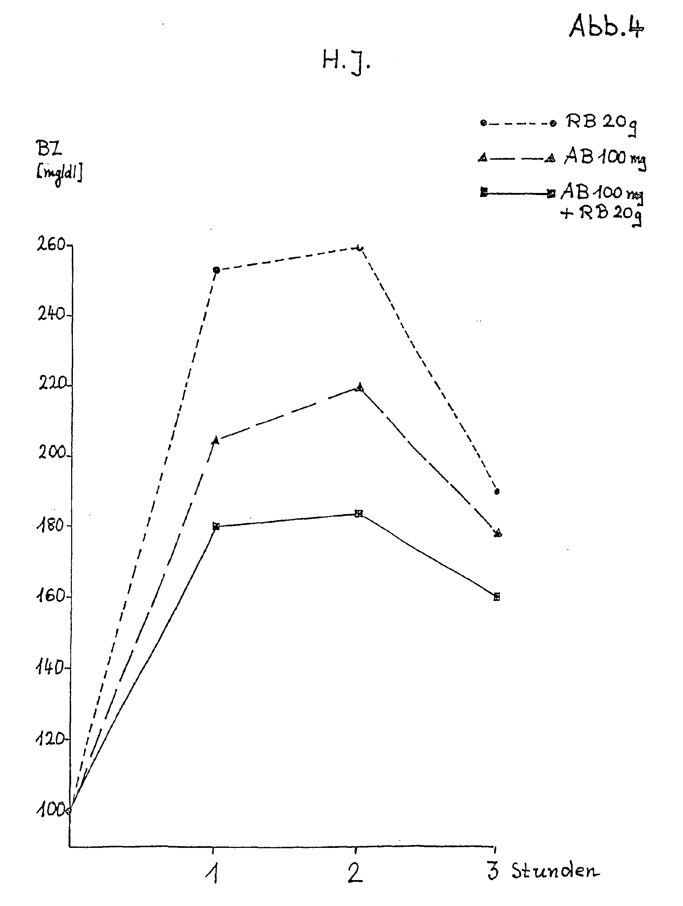

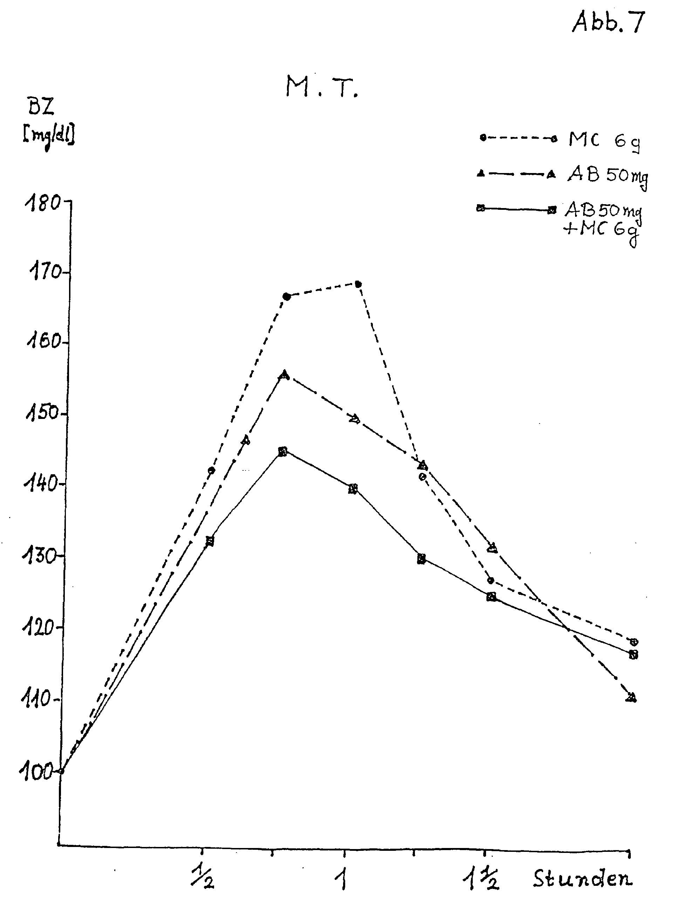

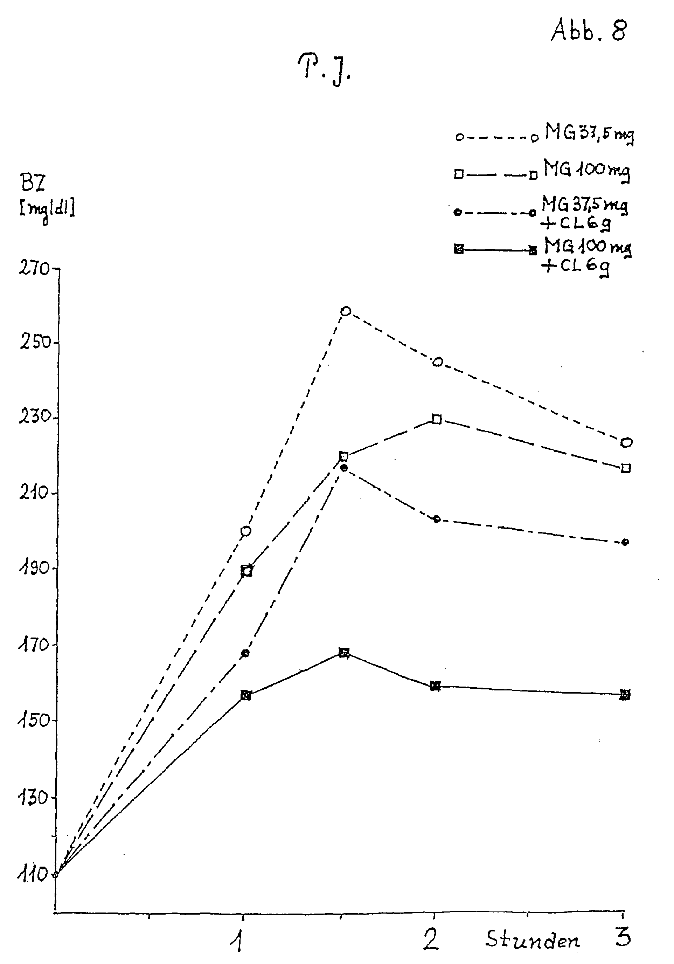

Typische Beispiele sind in den Abbildungen 4 bis 7 dokumentiert. Acarbose und Miglitol flachten den Blutzuckeranstieg nach dem Essen in bekannter Weise deutlich ab. Sie wirkten erheblich stärker als Ballaststoff. Eine Kombination von Glucosidasehemmern und Ballaststoff steigerte den Effekt der beiden Einzelsubstanzen relevant. Dieses Muster war bei 24 der 25 durchgeführten Versuchsserien mit quantitativen Variationen zu beobachten. Die Wirkungsverstärkung der Kombination gegenüber den Einzelsubstanzen war hochsignifikant (Tabelle 2). Durchschnittlich betrug die Senkung des postbrandialen Blutzuckeranstiegs 30,0+/- 8,9% durch Ballaststoff allein (a), 48,6+/- 17,9% durch Alpha-Glucosidasehemmer (b) und 66,8+/- 17,9% durch die Kombination (c). Die Differenzen zwischen (a) und (c) sowie zwischen (b) und (c) sind mit einer Irrtumswahrscheinlichkeit von jeweils p < 0.001 statistisch signifikant. Die Überlegenheit der Kombination wird noch deutlicher, wenn man die Bandbreite der Wirkung untersucht (Tabelle 4): Eine mehr als 50%ige Blutzuckersenkung war bei den Glucosidasehemmern nur in 18 von 35 Fällen, bei der Kombination aber in 33 der 35 Fälle zu beobachten. Eine Senkung um mehr als 80% wurde bei den Glucosidasehemmern in keinem Fall, bei der Kombination jedoch in 7 der 35 Experimente registriert. Bei Miglitol wurde neben der empfohlenen Standarddosis von 100 mg auch in 6 Fällen die Dosis 25 mg und 8mal die Dosis 50 mg untersucht (Tab. 2, Abb. 8). Die Wirkungsverstärkung des Miglitol durch eine Fixdosis von Ballaststoff trat auf jedem Dosisniveau ein. Durch Kombination mit Ballaststoff wurde die Wirkung von 25 mg Miglitol auf die Höhe von 100 mg ohne begleitenden Ballaststoff angehoben (Tab. 5). Die Kombination mit der niedrigen Dosis senkte den Blutzuckeranstieg um durchschnittlich 56%, die vierfach höhere Dosis allein bewirkte eine Senkung von 58%. In einigen Fällen wurden auch verschiedene Acarbosedosen untersucht. Dabei wirkten 25 mg plus Ballaststoff nur etwas schwächer als 100 mg allein, und 50 mg plus Ballaststoff übertrafen den Effekt von 100 mg allein. Unterschiede zwischen den Ballaststoffen Cellulose, Rübenballast und entfettetem Leinsamen bei der Wirkungsverstärkung der Glucosidasehemmer sind nicht erkennbar (Tab. 2). Lediglich bei der in der Tabelle nicht aufgeführten Methylcellulose scheint der Verstärkungseffekt etwas geringer zu sein (Abb. 7).

### Langzeitversuche mit Alpha-Glucosidasehemmern und Ballaststoffen

Bei 6 Patienten wurde während der Versuchsdauer täglich nach Protokoll eigenständig der Blutzucker morgens nüchtern und 2 Stunden nach individuellem Frühstück gemessen und registriert. Bei 2 über ein halbes Jahr behandelten Patienten wurden allmonatlich auch der HbA1C-Wert und als Stichprobe der 1-Stunden Wert des Blutzuckers nach dem Frühstück bestimmt. Auf eine mehrwöchige Kontrollphase unter Beibehaltung der Vormedikation erfolgten in mehrwöchigen Etappen die Testphasen mit den Zugaben von Ballaststoff und Glucosidasehemmer. Vor dem Frühstück, Mittag- und Abendessen wurden zuerst Acarbose 25-100 mg oder Miglitol (25-50 mg) und dann Rübenballaststoff (10 g) oder Cellulose (5 g) genommen.

Tabelle 6 fasst die Ergebnisse zusammen. In jedem Einzelfall verstärkten sich Ballaststoff und Acrabose/Miglitol bei Kombination. Im Durchschnitt sank nach 60 Tagen Kombinationsbehandlung der Nüchternblutzucker um 17 mg/dl und der Blutzuckeranstieg nach dem Essen um 49 mg/dl. Dieser 2-Stundenwert gibt wahrscheinlich die erreichte Besserung der Stoffwechsellage nur unzureichend wieder, weil (wie von den Kurzzeitversuchen bekannt) die postprandialen Blutzuckersenkungen nach 1 Stunde repräsentativer als nach 2 Stunden sind. Bei den erwähnten Halbjahresbeobachtungen von 2 anderweitig unbehandelten Patienten verringerte sich der Blutzuckeranstieg 1 Stunde nach dem Frühstück um schließlich 69 bzw. 91 mg/dl, und die HbA1C-Werte nahmen um 1,3% ab (Tab. 7).

### Nebenwirkungen der Kombinationsbehandlung

Bei den Kurzzeitversuchen mit 100 mg Acrabose allein gaben 3 von 9 Patienten mehrstündige abdominale Beschwerden an. Im Fall von Miglitol traten bei 4 von 9 Patienten breiige Stühle bis zu Durchfällen auf. Bei 50 mg Acarbose (2 Fälle) und 50 mg Miglitol (8 Fälle) bzw. 25 mg Miglitol (5 Fälle) waren keine eindeutigen Begleiterscheinungen festzustellen. Bei allen Kombinationen von Ballaststoff mit Acarbose oder Miglitol, auch bei den 100 mg-Dosen, traten in den Kurzzeitversuchen keine Nebenwirkungen auf. Wenn Normalpersonen statt des Testfrühstücks 50-75 g Saccharose erhielten, traten nach 100 mg Acarbose bzw. Miglitol Bauchschmerzen und Durchfälle auf. Diese Erscheinungen wurden bei Kombination des Glucosidasehemmers mit Rübenballaststoff oder mit Cellulose deutlich gemildert. Bei den Langzeitbeobachtungen gaben 4 der 6 Patienten unter Glucosidasehemmern, besonders nach 3mal täglich 100 mg, Bauchbeschwerden und Blähsucht an. Durch Zugabe von Rübenballast oder Cellulose vergingen die Beschwerden, waren aber bei einer Patientin bei 3mal 50 mg Miglitol noch 14 Tage in milder Form vorhanden. In ergänzenden Untersuchungen erhielten je 5 Versuchspersonen 2 Tage 3mal 50 mg Acarbose bzw. Miglitol plus 5 g Cellulose. Es traten keine unerwünschten Erscheinungen auf.
Wa rum unlösliche Ballastfasern intestinalen Begleiteffekte der Alpha-Glucosidasehemmer herabsetzen, kann nur vermutet werden: Einerseits reduzieren unlösliche Ballastfasern die Symptome gesteigerter Darmmotorik, andererseits werden durch die Amylasehemmung weniger Disaccharide gebildet, die im Dickdarm zu Irritationen führen würden.

### Zusammenfassung und Bewertung der Kombinationsversuche

Durch die Kombination von Alpha-Glucosidasehemmern und unlöslichen Ballaststoffen wird ein wesentlicher Fortschritt in der Therapie des Typ II-Diabetes erzielt. Die Wirkung der Glucosidasehemmer wird um mehr als ein Drittel gesteigert, sodass der Blutzuckeranstieg nach dem Essen durchschnittlich um fast 70% reduziert wird. Dies bedeutet im Einzelfall z.B. bei einem Diabetiker mit einem Nüchternblutzucker von 120 mg/dl und einem maximalen postprandialen Blutzuckeranstieg auf 220 mg/dl, dass dieser durch die Kombinationstherapie auf 150 mg/dl verringert wird. Das ist ein Wert im oberen Normbereich.

Da der blutzuckersenkende Effekt auf einer Resorptionshemmung für Kohlenhydrate im Dünndarm beruht, spart der behandelte Diabetiker auf diese Weise Insulin, seine Insulinproduktion erholt sich, und die Insulinresistenz nimmt ab, wie in den Langzeitversuchen am abnehmenden morgendlichen Nüchternzucker ablesbar ist. Dieser äußerst positiven Beeinflussung des Krankheitsverlaufs stehen keine relevanten Nebenwirkungen (wie bei den anderen Antidiabetica) gegenüber. Die Häufigkeit unerwünschter Begleiteffekte bei alleiniger Glucosidasetherapie geht mit der Kombination von etwa 50% auf 5% zurück. Aufgrund der in den meisten Fällen möglichen Dosisreduzierung von 100 auf 50 mg wird die Intensität dieser Begleiterscheinungen zusätzlich abgeschwächt, sodass sie von den wenigen betroffenen Patienten problemlos toleriert werden.

Unter dem Gesichtspunkt der therapeutischen Praktikabilität ist Cellulose der ideale Ballaststoff. Sie ist als Reinsubstanz verfügbar, leicht in Flüssigkeiten suspendierbar, geschmacksneutral, in problemlos schluckbaren Mengen von 3-7 g wirksam und obendrein preiswert. Nach einhelliger Meinung führender Diabetologen ist ein Europa und in der USA etwa die Hälfte der Typ II-Diabetiker mit einer Tablettentherapie unzureichend eingestellt. Mit der erfindungsgemäßen neuen Therapie könnten die meisten dieser Patienten optimal behandelt werden.

**Tabelle 1 Ballaststoffgehalt pro 100 g der untersuchten Ballaststoffpräparate (laut Herstellerangaben) ***

| | Cellulose | Hemicellulose | Lignin | Pektin |
|---|---|---|---|---|
| Rübenballast | 24 g | 24 g | 4 g | 31 g |
| Leinsamen ** | 7 g | 13 g | 3 g | 24 g |
| Weizenkleie | 21 g | 22 g | 8 g | -- -- |
| Cellulose | 99 g | -- -- | -- -- | -- -- |
| Methylcellulose | (99 g)*** | -- -- | -- -- | -- -- |

| | | | | |
|---|---|---|---|---|
| * Der Rest besteht aus Eiweiß, Fett, Zucker und Mineralien | | | | |
| ** Durch Acetonbehandlung wurde der Fettgehalt des verwendeten Leinsamenschrots von 40 auf 14 % reduziert. | | | | |
| *** Künstlich veränderte Cellulose | | | | |

**Tabelle 2 Minderung des Blutzuckeranstiegs nach Testfrühstück durch die Ballaststoffe Rübenballast (RB), Leinsamen (LS) und Cellulose (CL) oder/und die Alpha-Glukosidasehemmer Acarbose (AB) und Miglitol(MG)**

| | Behandlung | | Blutiuckersenkung | | |
|---|---|---|---|---|---|
| Patient | Ballast | Alpha-GH | Ballast | Alpha-GH | Ballast + Alpha-GH |
| H.J. | RB 20g | AB 100mg | 18 % | 41 % | 57 % |
| S.W.(n*) | RB 20g | AB 100mg | 40 % | 63 % | 81 % |
| S.W.(zf**) | RB 20g | AB 100mg | 45 % | 56 % | 78 % |
| T.W. | FB 20g | AB 100mg | 46 % | 65 % | 84 % |
| J.K.(n*) | RB 20g | AB 100mg | 4 % | 6 % | 65 % |
| J.K.(zf**) | RB 20g | AB 100mg | 27 % | 55 % | 60 % |
| F.J. | RB 20g | AB 100mg | 17 % | 39 % | 60 % |
| H.E. | RB 20g | AB 50mg | 31 % | 42 % | 50 % |
| F.J. | RB 20g | MG 100mg | 19 % | 24 % | 53 % |
| S.A. | RB 20g | MG 100mg | 40 % | 60 % | 82 % |
| W.U. | LS 15g | AB 100mg | 28 % | 63 % | 68 % |
| S.W. | LS 15g | AB 100mg | 51 % | 58 % | 60 % |
| K.F. | LS 15g | MG 50mg | 24 % | 33 % | 42 % |
| S.A. | LS 15g | MG 25mg | 46 % | 27 % | 68 % |
| S.A. | LS 15g | MG 100mg | 46 % | 80 % | 90 % |
| A.C. | CL 6g | AB 50mg | 28 % | 50 % | 76 % |
| A.C. | CL 6g | AB 100mg | 28 % | 59 % | 82 % |
| S.M. | CL 6g | MG 50mg | 19 % | 40 % | 75 % |
| W.H. | CL 6g | MG 50mg | 17 % | 37 % | 50 % |
| M.S. | CL 6g | MG 50mg | 28 % | 62 % | 73 % |
| S.W. | CL 7g | MG 25mg | 33 % | 47 % | 70 % |
| S.W. | CL 7g | MG 50mg | 33 % | 62 % | 74 % |
| S.W. | CL 7g | MG 100mg | 33 % | 59 % | 93 % |
| P.J. | CL 6g | MG 25mg | 22 % | 12 % | 35 % |
| P.J. | CL 6g | MG 50mg | 22 % | 25 % | 57 % |
| P.J. | CL 6g | MG 100mg | 22 % | 32 % | 64 % |
| B.A. | CL 6g | MG 25mg | 36 % | 53 % | 60 % |
| B.A. | CL 6g | MG 50mg | 36 % | 59 % | 69 % |
| B.A. | CL 6g | MG 100mg | 36 % | 66 % | 74 % |
| S.A. | CL 6g | MG 25mg | 30 % | 49 % | 70 % |
| S.A. | CL 6g | MG 100mg | 30 % | 75 % | 89 % |
| F.J. | CL 6g | MG 25mg | 27 % | 24 % | 35 % |
| F.J. | CL 6g | MG 100mg | 27 % | 34 % | 63 % |
| B.W. | CL 6g | MG 50mg | 30 % | 71 % | 70 % |
| B.W. | CL 6g | MG 100mg | 30 % | 73 % | 58 % |
| Mittelwerte | | | 30,0 % | 48,6 % | 66,8 % |
| | | | ±8.9 | ±17,9 | ±15,0 |

| | | | | | |
|---|---|---|---|---|---|
| * n = normales Testfrühstück | | | | | |
| ** zf= zuckerfreies Testfrühstück | | | | | |

**Tabelle 3 Langzeiteffekt von Rübenballaststoff und Cellulose**

| Patient. | Begleittherapie | vor BS* (Kontrolle) | | BS | nach BS Dauer Blutzucker (mg/dl) | | |
|---|---|---|---|---|---|---|---|
| | | NBZ** | ppBZ*** | | (Tage) | NBZ | ppBZ |
| KP. | - | - | 135 | RB | 120 | - | 104 |
| B.R. | - | - | 159 | RB | 120 | - | 105 |
| H.E. | - | 145 | 164 | RB | 13 | 137 | 138 |
| S.E. | - | 122 | 181 | RB | 22 | 120 | 151 |
| G.W. | GB, MF | 139 | 175 | RB | 28 | 134 | 110 |
| G.T. | GP | - | 161 | RB | 14 | - | 106 |
| P.E. | GP | 133 | 155 | RB | 15 | 131 | 119 |
| H.J. | Insulin | 164 | 216 | RB | 10 | 158 | 192 |
| S.H. | GP,MF | 181 | 200 | RB | 7 | 182 | 177 |
| L.S. | - | 140 | 185 | CL | 14 | 136 | 170 |
| G.R. | MG | 118 | 175 | CL | 42 | 116 | 148 |
| | | | | | | | |
| Mittelwerte | | 143 | 173 | | | 139 | 138 |

| | | | | | | | |
|---|---|---|---|---|---|---|---|
| * BS = Ballaststoff | | | | | | | |
| ** NBZ = Nüchternblutzucker | | | | | | | |
| ***ppBZ = Blutzucker 2 Stunden nach dem Frühstück | | | | | | | |
| GB = Glibornurid | | | | | | | |
| GP = Glimepirid | | | | | | | |
| MF = Metformin | | | | | | | |
| MG = Miglitol | | | | | | | |
| RB = Rübenballaststoff | | | | | | | |
| CL = Cellulose | | | | | | | |

**Tabelle 4 Effektivität der Alpha-Glukosidasehemmer. Vergleich der Monotherapie mit der Kombinationstherapie von Glucosidasehemmer plus Ballaststoff. Ergebnis von 35 Kurzzeitversuchen.**

| Minderung des postprandialen Blutzuckers | Alpha-GH* | Alpha-GH plus BS** |
|---|---|---|
| >40% | 23 | 34 |
| >50% | 18 | 33 |
| >60% | 10 | 23 |
| > 70 % | 4 | 13 |
| > 80 % | 0 | 7 |

| | | |
|---|---|---|
| * Alpha-GH = Alpha-glucosidasehemmer | | |
| ** BS = Ballaststoff | | |

**Tabelle 5 Wirkungsvergleich von Miglitol 25 mg plus Ballasfstoff und Miglitol 100 mg allein**

| Patient | Ballaststoff | Blutzuckersenkung | |
|---|---|---|---|
| | | MG 25 plus BS | MG 100 |
| S.A. | LS | 68% | 80% |
| S.W. | CL | 70% | 59% |
| P.J. | CL | 35 % | 32 % |
| B.A. | CL | 60 % | 66 % |
| S.A. | CL | 70 % | 75 % |
| F.J. | CL | 35 % | 34 % |
| | | | |
| Mittelwerte | | 56,3 % | 57,7 % |

| | | | |
|---|---|---|---|
| MG = Miglitol | | | |
| BS = Ballaststoff | | | |
| LS = Leinsamen | | | |
| CL = Cellulose | | | |

**Tabelle 6 Langzeiteffekt der Kombination von Ballaststoff mit Alpha-Glukosidasehemmem**

| Pat. | Begleittherapie | Kontrolle | | | BS plus Alpha-GH | | | |
|---|---|---|---|---|---|---|---|---|
| | | nBZ | ppBZ | BS | Alpha-GH | Dauer (Tage) | nBZ* | ppBZ** |
| H.J. | Insulin | 164 | 216 | RB 10 g | AB 100 | 14 | 151 | 167 |
| P.E. | GP | 133 | 155 | RB 10 g | AB 50 | 14 | 130 | 107 |
| S.H. | GP,MF | 187 | 200 | RB 10 g | AB 50 | 8 | 166 | 162 |
| | | 187 | 200 | RB 10 g | AB 100 | 14 | 147 | 142 |
| G.R. | - | 118 | 175 | CL 5 g | MG 50 | 42 | 116 | 148 |
| L.S. | - | 140 | 185 | CL 5g | MG 50 | 150 | 115 | 126 |
| A.C. | - | 128 | 168 | CL5g | AB 25 | 178 | 110 | 104 |
| | | | | | | | | |
| Mittelwerte | | 151 | 186 | | | 60 | 134 | 137 |

| | | | | | | | | |
|---|---|---|---|---|---|---|---|---|
| *nBZ = Nüchternblutzucker (mg/dl) | | | | | | | | |
| **ppBZ = Blutzucker 2 Stunden nach dem Frühstück (mg/dl) | | | | | | | | |
| BS = Ballaststoff | | | | | | | | |
| Alpha-GH = Alpha-Glucosidasehemmer | | | | | | | | |
| GP = Glimepirid | | | | | | | | |
| MF = Metformin | | | | | | | | |
| RB = Rübenballaststoff | | | | | | | | |
| CL = Cellulose | | | | | | | | |
| AB 50,100 = Acarbose 50 u. 100 mg | | | | | | | | |
| MG 25,50 =Miglitol 25 u. 50 mg | | | | | | | | |

**Tabelle 7 Sechsmonatige Langzeittherapie mit Cellulose (CL) plus Acarbose (AB) oder Miglitol (MG)**

| | A.C.* | | L.S.** | |
|---|---|---|---|---|
| | pp.BZ*** (mg/dl) | HbA1C (%) | pp.BZ*** (mg/dl) | HbA1C (%) |
| vor Therapie | 194 | 7,1 | 222 | 7,9 |
| nach 1 Monat | 140 | 6,9 | 161 | 7,8 |
| nach 3 Monaten | 132 | 6,1 | 146 | 7,0 |
| nach 5-6 Monaten | 125 | 5,8 | 131 | 6,6 |

| | | | | |
|---|---|---|---|---|
| * 3 x 25 mg AB + CL (6 Monate) | | | | |
| ** 3 x 50 mg MG + CL (5,5 Monate) | | | | |
| ***postprandialer Blutzucker 1 Stunde nach dem Frühstück (Mittelwert von 3 Tagen) | | | | |

### Legenden der Abbildungen 1 - 8

- Abb. 1 :: Rübenballaststoff-Effekl.
H.E., Diabetes Typ-II, 64 J, sonst mit Glimepirid (Sulfonylharnstoffpräparat) behandelt.
Blutzuckerkurve nach Testfrühstück und 10 bzw. 20 g Rübenballaststoff (RB). Kontrollkurve ohne Ballaststoff. Der Nüchtern-Blutzucker ist auf 110 mg/dl (Meßwerte 106 -115 mg/dl).
- Abb.2:: Leinsamen-Effekt.
S.W., Diabetes Typ-II, 67 J, unbehandelt.
Blutzuckerkurven nach Testfrühstück und 10 bzw. 20 g entfettetem Leinsamen (LS). Der Nüchtern-Blutzucker ist auf 120 mg/dl normiert (Meßwerte 112 - 124 mg/dl)
- Abb.3:: Cellulose-Effekt.
S.W., Diabetes Typ-II, 67 J, unbehandelt.
Blutzuckerkurven nach Testfrühstück und 5 bzw. 7 g Cellulose (CL).
- Abb. 4 :: Kombinations-Effekt von Rübenballaststoff und Acarbose.
H.J., Diabetes Typ-II, 59 J, sonst mit Insulin behandelt.
Blutzuckerkurven nach 20 g Rübenballast (RB), 100 mg Acarbose (AB) und Acarbose plus Rübenballast (AB+RB).
Der Nüchtern-Blutzucker ist auf 100 mg/dl normiert (Meßwerte 98 -114 mg/dl).
- Abb. 5 :: Kombinations-Effekt von Leinsamen und Miglitol.
S.A., Diabetes Typ-II, 67 J, sonst mit Glibenclamid behandelt.
Blutzuckerkurven nach 15 g entfettetem Leinsamen (LS), 50 mg Miglitol (MG) und Miglitol plus Leinsamen (MG+LS).
Der Nüchtern-Blutzucker ist auf 130 mg/dl normiert (Meßwerte 122 - 135 mg/dl).
- Abb. 6 :: Kombinations-Effekt von Cellulose und Acarbose.
A.C., Diabetes Typ-II, 60 J, unbehandelt.
Blutzuckerkurven nach 6 g Cellulose (CL), 50 mg Acarbose (AB) und Acarbose plus Cellulose (AB+CL)
Der Nüchtern-Blutzucker ist auf 120 mg/dl normiert (Meßwerte 113 -125 mg/dl).
- Abb. 7 :: Kombinations-Effekt von Methylcellulose und Acarbose.
M.T., Diabetes Typ-II, 67 J, unbehandelt.
Blutzuckerkurven nach 6 g Methylcellulose (MC), 50 mg Acarbose (AB) und Acarbose plus Methylcellulose (AB+MC).
Der Nüchtern-Blutzucker ist auf 100 mg/dl normiert (Meßwerte 100 -113 mg/dl).
- Abb. 8 :: Kombinations-Effekt mit verschiedenen Dosen eines Glucosidasehemmers.
P.J., Diabetes Typ-II, 59 J, sonst mit Insulin behandelt.
Blutzuckerkurven nach Einzeldosen von 37,5 mg bzw. 100 mg Miglitol (MG) und nach der Kombination von 37,5 mg MG mit 6 g Cellulose (MG 37,5 mg + CL) oder 100 mg Miglitol mit 6 g Cellulose (MG 100 mg + CL).
Der Nüchtern-Blutzucker ist auf 110 mg/dl normiert (Meßwerte 102 - 126 mg/dl).

## Patentansprüche

1. Mittel zur Behandlung des Diabetes mellitus, **dadurch gekennzeichnet, dass** es wenigstens einen pflanzlichen Ballaststoff mit einem hohen Anteil unlöslicher Faserstoffe umfasst in Kombination mit wenigstens einem Alpha-Glucosidasehemmer.

2. Mittel nach Anspruch 1 zur Behandlung des Diabetes mellitus Typ II.

3. Mittel nach einem der Ansprüche 1 oder 2, **dadurch gekennzeichnet, dass** der Ballaststoff einen Anteil von vorzugsweise mehr als 20 g pro 100 g unlösliche Faserstoffe enthält.

4. Mittel nach einem der Ansprüche 1 bis 3, **dadurch gekennzeichnet, dass** es pro Einzeldosis etwa 2,5 g bis etwa 10 g unlösliche Fasern enthält.

5. Mittel zur Behandlung des Diabetes mellitus, **dadurch gekennzeichnet, dass** es wenigstens einen pflanzlichen Ballaststoff mit einem hohen Anteil unlöslicher Faserstoffe enthält zur Einnahme unmittelbar vor oder nach der Einnahme eines einen Alpha-Glucosidasehemmer enthaltenden Mittels.

6. Mittel nach einem der Ansprüche 1 bis 5, **dadurch gekennzeichnet, dass** es wenigstens einen Alpha-Glucosidasehemmer, vorzugsweise ausgewählt aus Acarbose, Miglitol, Voglibose und Emiglitat enthält.

7. Mittel nach einem der Ansprüche 1 bis 6, **dadurch gekennzeichnet, dass** es wenigstens einen Alpha-Glucosidasehemmer ausgewählt aus Acarbose und Miglitol in einer Menge von zwischen etwa 20 und etwa 200 mg enthält.

8. Mittel nach einem der Ansprüche 1 bis 7, **dadurch gekennzeichnet, dass** wenigstens einer der enthaltenen Ballaststoffe ein pflanzlicher Ballaststoff ist, der aus einer Zuckerfrucht gewonnen wurde.

9. Mittel nach einem der Ansprüche 1 bis 8, **dadurch gekennzeichnet, dass** wenigstens einer der Ballaststoffe ein Ballaststoff ist, der aus einer Rübenfrucht gewonnen wurde.

10. Mittel nach einem der Ansprüche 1 bis 9, **dadurch gekennzeichnet, dass** mindestens ein Rübenballaststoff enthaltendes Mittel in Form von Granulat, Pulver, Keks oder als dragiertes Granulat vorliegt.

11. Mittel nach Anspruch 10, **dadurch gekennzeichnet, dass** es Acarbose und/oder Miglitol bzw. andere Alphaglucosidasehemmer umfasst, die vorzugsweise in Tablettenform vorliegen.

12. Mittel nach einem der Ansprüche 1 bis 11, **dadurch gekennzeichnet, dass** dieses Rübenballaststoff in einer Menge von etwa 5 g bis etwa 30 g vorzugsweise von etwa 10 g bis etwa 20 g enthält.

13. Mittel nach einem der Ansprüche 1 bis 12, **dadurch gekennzeichnet, dass** dieses als Ballaststoffe Cellulose, Hemicellulose (Pentosane) und Lignin enthält.

14. Mittel zur Behandlung des Diabetes mellitus, **dadurch gekennzeichnet, dass** es Cellulose umfasst in Kombination mit wenigstens einem Alpha-Glucosidasehemmer.

15. Mittel nach Anspruch 14, **dadurch gekennzeichnet, dass** die Cellulose einerseits und der Alpha-Glucosidasehemmer andererseits in getrennten Präparaten vorliegen.

16. Mittel nach Anspruch 14 oder 15, **dadurch gekennzeichnet, dass** ein Cellulose enthaltendes Präparat zur Einnahme in nahem zeitlichen Zusammenhang nach der Einnahme eines einen Alpha-Glucosidasehemmer enthaltenden Mittels bestimmt ist.

17. Mittel nach einem der Ansprüche 14 bis 16, **dadurch gekennzeichnet, dass** es als Kombinationspräparat konfektioniert ist, wobei ein erstes Präparat Cellulose und ein zweites Präparat wenigstens einen Alpha-Glucosidasehemmer enthält.

18. Mittel nach einem der Ansprüche 14 bis 17, **dadurch gekennzeichnet, dass** die Cellulose in Pulverform vorliegt.

19. Mittel nach einem der Ansprüche 14 bis 18, **dadurch gekennzeichnet, dass** das die Cellulose enthaltende Präparat als Pulver oder Kautablette zusammen mit einem Bindemittel konfektioniert ist.

20. Mittel nach einem der Ansprüche 14 bis 19, **dadurch gekennzeichnet, dass** es pro Dosis eine Menge von etwa 1 bis etwa 10 g Cellulose enthält.

21. Mittel nach einem der Ansprüche 14 bis 20, **dadurch gekennzeichnet, dass** es pro Dosis wenigstens einen Alpha-Glucosidasehemmer ausgewählt aus Acarbose und Miglitol in einer Menge von zwischen etwa 10 und etwa 200 mg enthält.

22. Mittel nach einem der Ansprüche 14 bis 21, **dadurch gekennzeichnet, dass** es wenigstens einen Alpha-Glucosidasehemmer, vorzugsweise ausgewählt aus Acarbose, Miglitol, Voglibose und Emiglitat enthält.

23. Mittel nach einem der Ansprüche 14 bis 22, **dadurch gekennzeichnet, dass** es pro Dosis eine Menge von etwa 3 bis 10 g Cellulose enthält.

24. Mittel zur Behandlung des Diabetes mellitus des Menschen, insbesondere des Diabetes Typ II, **dadurch gekennzeichnet, dass** dieses Cellulose pro Dosis enthält in einer Menge von etwa 3 bis 10 g.

25. Mittel nach Anspruch 24, **dadurch gekennzeichnet, dass** dieses Cellulose im Wesentlichen in reiner Form enthält.

26. Mittel nach Anspruch 24 oder 25, **dadurch gekennzeichnet, dass** dieses ein Cellulosederivat und wenigstens einen Alpha-Glucosidasehemmer umfasst, welche in Kombination in einem Präparat oder jeweils in getrennten Präparaten vorliegen können.

27. Mittel nach einem der Ansprüche 1 bis 26, **dadurch gekennzeichnet, dass** dieses entfetteten Leinsamen vorzugsweise in einer Dosis von 10 g - 20 g als Ballaststoff enthält.

28. Mittel nach einem der Ansprüche 1 bis 27, **dadurch gekennzeichnet, dass** mindestens der Ballaststoff zur Einnahme unmittelbar vor oder zu einer Mahlzeit bestimmt ist.

## Claims

1. Composition for the treatment of diabetes mellitus, **characterized in that** it comprises at least one vegetable bulking agent with a high proportion of insoluble fibres, in combination with at least one alpha-glucosidase inhibitor.

2. Composition according to Claim 1 for the treatment of type II diabetes mellitus.

3. Composition according to either of Claims 1 or 2, **characterized in that** the bulking agent comprises a proportion of insoluble fibres of preferably more than 20g per 100g.

4. Composition according to any of Claims 1 to 3, **characterized in that** it comprises about 2.5 g to about 10 g of insoluble fibres per single dose.

5. Composition for the treatment of diabetes mellitus, **characterized in that** it comprises at least one vegetable bulking agent with a high proportion of insoluble fibres for intake immediately before or after the intake of a composition comprising an alpha-glucosidase inhibitor.

6. Composition according to any of Claims 1 to 5, **characterized in that** it comprises at least one alpha-glucosidase inhibitor, preferably selected from acarbose, miglitol, voglibose and emiglitate.

7. Composition according to any of Claims 1 to 6, **characterized in that** it comprises at least one alpha-glucosidase inhibitor selected from acarbose and miglitol in an amount of between about 20 and about 200 mg.

8. Composition according to any of Claims 1 to 7, **characterized in that** at least one of the bulking agents present is a vegetable bulking agent which has been obtained from a sacchariferous fruit.

9. Composition according to any of Claims 1 to 8, **characterized in that** at least one of the bulking agents is a bulking agent which has been obtained from a beet fruit.

10. Composition according to any of Claims 1 to 9, **characterized in that** at least one composition comprising beet bulking agent is in the form of granules, powder, biscuits or as coated granules.

11. Composition according to Claim 10, **characterized in that** it comprises acarbose and/or miglitol or other alpha-glucosidase inhibitors which are preferably in tablet form.

12. Composition according to any of Claims 1 to 11, **characterized in that** the latter comprises beet bulking agent in an amount of about 5 g to about 30 g, preferably of about 10 g to about 20 g.

13. Composition according to any of Claims 1 to 12, **characterized in that** the latter comprises cellulose, hemicellulose (pentosans) and lignin as bulking agents.

14. Composition for the treatment of diabetes mellitus, **characterized in that** it comprises cellulose in combination with at least one alpha-glucosidase inhibitor.

15. Composition according to Claim 14, **characterized in that** the cellulose on the one hand and the alpha-glucosidase inhibitor on the other hand are present in separate products.

16. Composition according to Claim 14 or 15, **characterized in that** a cellulose-containing product is intended for intake in close temporal association after the intake of a composition comprising an alpha-glucosidase inhibitor.

17. Composition according to any of Claims 14 to 16, **characterized in that** it is formulated as combination product, where a first product comprises cellulose and a second product comprises at least one alpha-glucosidase inhibitor.

18. Composition according to any of Claims 14 to 17, **characterized in that** the cellulose is present in powder form.

19. Composition according to any of Claims 14 to 18, **characterized in that** the cellulose-containing product is formulated as powder or chewable tablet together with a binder.

20. Composition according to any of Claims 14 to 19, **characterized in that** it comprises an amount of about 1 to about 10 g of cellulose per dose.

21. Composition according to any of Claims 14 to 20, **characterized in that** it comprises at least one alpha-glucosidase inhibiter selected from acarbose and miglitol in an amount of between 10 and about 200 mg per dose.

22. Composition according to any of Claims 14 to 21, **characterized in that** it comprises at least one alpha-glucosidase inhibitor preferably selected from acarbose, miglitol, voglibose and emiglitate.

23. Composition according to any of Claims 14 to 22, **characterized in that** it comprises an amount of about 3 to 10 g of cellulose per dose.

24. Composition for the treatment of diabetes mellitus in humans, in particular of type II diabetes, **characterized in that** the latter comprises cellulose in an amount of about 3 to 10 g per dose.

25. Composition according Claim 24, **characterized in that** the latter comprises cellulose in the substantially pure form.

26. Composition according to Claim 24 or 25, **characterized in that** the latter comprises a cellulose derivative and at least one alpha-glucosidase inhibitor, which may be present in combination in one product or each in separate products.

27. Composition according to any of Claims 1 to 26, **characterized in that** the latter comprises defatted linseed preferably in a dose of 10 g-20 g as bulking agent.

28. Composition according to any of Claims 1 to 27, **characterized in that** at least the bulking agent is intended for intake directly before or during a meal.

## Revendications

1. Composition pour le traitement du diabète sucré, **caractérisée en ce qu'**elle comprend au moins une matière inerte végétale ayant une forte teneur en fibres insolubles, en association avec au moins un inhibiteur d'α-glucosidase.

2. Composition selon la revendication 1, destinée au traitement du diabète sucré de type II.

3. Composition selon la revendication 1 ou 2, **caractérisée en ce que** la matière inerte contient de préférence plus de 20 g de matières fibreuses insolubles par 100 g.

4. Composition selon l'une quelconque des revendications 1 à 3, **caractérisée en ce qu'**elle contient par dose unitaire environ 2,5 g à environ 10 g de fibres insolubles.

5. Composition destinée au traitement du diabète sucré, **caractérisée en ce qu'**elle contient au moins une matière inerte végétale ayant une forte teneur en matières fibreuses insolubles, pour la prise immédiatement avant ou après la prise d'un agent contenant un inhibiteur d'α-glucosidase.

6. Composition selon l'une quelconque des revendications 1 à 5, **caractérisée en ce qu'**elle contient au moins un inhibiteur d'α-glucosidase, de préférence choisi parmi l'acarbose, le miglitol, le voglibose et l'émiglitate.

7. Composition selon l'une quelconque des revendications 1 à 6, **caractérisée en ce qu'**elle contient au moins un inhibiteur d'α-glucosidase, choisi parmi l'acarbose et le miglitol, en une quantité comprise entre environ 20 et environ 200 mg.

8. Composition selon l'une quelconque des revendications 1 à 7, **caractérisée en ce qu'**au moins l'une des matières inertes contenues est une matière inerte végétale qui a été obtenue à partir d'un fruit sucré.

9. Composition selon l'une quelconque des revendications 1 à 8, **caractérisée en ce qu'**au moins l'une des matières inertes est une matière inerte qui a été obtenue à partir d'une betterave.

10. Composition selon l'une quelconque des revendications 1 à 9, **caractérisée en ce qu'**au moins une composition contenant de la matière inerte de betterave est présente sous forme de produit granulé, poudre, biscuit ou sous forme de produit granulé dragéifié.

11. Composition selon la revendication 10, **caractérisée en ce qu'**elle comprend de l'acarbose et/ou du miglitol ou d'autres inhibiteurs d'α-glucosidase qui sont présents de préférence sous forme de comprimé.

12. Composition selon l'une quelconque des revendications 1 à 11, **caractérisée en ce qu'**elle contient la matière inerte de betterave en une quantité d'environ 5 g à environ 30 g, de préférence d'environ 10 g à environ 20 g.

13. Composition selon l'une quelconque des revendications 1 à 12, **caractérisée en ce qu'**elle contient en tant que matières inertes de la cellulose, de l'hémicellulose (pentosane) et de la lignine.

14. Composition destinée au traitement du diabète sucré, **caractérisée en ce qu'**elle comprend de la cellulose en association avec au moins un inhibiteur d'α-glucosidase.

15. Composition selon la revendication 14, **caractérisée en ce que** la cellulose d'une part et l'inhibiteur d'α-glucosidase d'autre part sont présents dans des préparations séparées.

16. Composition selon la revendication 14 ou 15, **caractérisée en ce qu'**une préparation contenant de la cellulose est destinée à la prise en proche relation temporelle après la prise d'une composition contenant un inhibiteur d'α-glucosidase.

17. Composition selon l'une quelconque des revendications 14 à 16, **caractérisée en ce qu'**elle est présentée sous forme d'une préparation consistant en une association de substances, une première préparation contenant de la cellulose et une seconde préparation contenant au moins un inhibiteur d'α-glucosidase.

18. Composition selon l'une quelconque des revendications 14 à 17, **caractérisée en ce que** la cellulose est présente sous forme de poudre.

19. Composition selon l'une quelconque des revendications 14 à 18, **caractérisée en ce que** la préparation contenant de la cellulose est présentée sous forme de poudre ou de comprimé à mâcher, conjointement avec un liant.

20. Composition selon l'une quelconque des revendications 14 à 19, **caractérisée en ce qu'**elle contient par dose une quantité d'environ 1 à environ 10 g de cellulose.

21. Composition selon l'une quelconque des revendications 14 à 20, **caractérisée en ce qu'**elle contient par dose au moins un inhibiteur d'α-glucosidase choisi parmi l'acarbose et le miglitol en une quantité comprise entre environ 10 et environ 200 mg.

22. Composition selon l'une quelconque des revendications 14 à 21, **caractérisée en ce qu'**elle contient au moins un inhibiteur d'α-glucosidase, de préférence choisi parmi l'acarbose, le miglitol, le voglibose et l'émiglitate.

23. Composition selon l'une quelconque des revendications 14 à 22, **caractérisée en ce qu'**elle contient par dose une quantité d'environ 3 à 10 g de cellulose.

24. Composition destinée au traitement du diabète sucré de l'homme, en particulier du diabète de type II, **caractérisée en ce qu'**elle contient par dose de la cellulose en une quantité d'environ 3 à 10 g.

25. Composition selon la revendication 24, **caractérisée en ce qu'**elle contient de la cellulose pratiquement sous forme pure.

26. Composition selon la revendication 24 ou 25, **caractérisée en ce qu'**elle comprend un dérivé de cellulose et au moins un inhibiteur d'α-glucosidase, qui peuvent être présents en association dans une préparation ou chacun dans des préparations distinctes.

27. Composition selon l'une quelconque des revendications 1 à 26, **caractérisée en ce qu'**elle contient comme matière inerte des graines de lin dégraissées, de préférence à une dose de 10 g - 20 g.

28. Composition selon l'une quelconque des revendications 1 à 27, **caractérisée en ce qu'**au moins la matière inerte est destinée à la prise immédiatement avant ou pendant un repas.
